# EUROPÄISCHE PATENTANMELDUNG

(11) **EP 2 447 016 A1**
(43) Veröffentlichungstag der Anmeldung: **02.05.2012**
(21) Anmeldenummer: 11182707.7
(22) Anmeldetag: 26.09.2011
(51) Int. Cl.: B25J 19/00, A61B 6/00

(54) **Roboteranordnung mit einem Führungselement für Versorgungsleitungen**

(30) Priorität: 29.10.2010 DE 102010043121
(71) Anmelder: Siemens Aktiengesellschaft, 80333 Munich (DE)
(72) Erfinder: Neuber, Wolfgang, 92690 Pressath (DE)

(57) **Zusammenfassung**

Die vorliegende Erfindung betrifft eine Anordnung mit einem Roboter (1) mit einem aus Armgliedern (6) gebildeten Roboterarm (5), der ein von mindestens einer elektrischen Versorgungsleitung gespeistes Gerät (3,4) trägt. Die Anordnung weist mindestens ein am Armglied (6) befestigtes Führungselement (10) zur Führung der Versorgungsleitung am Roboterarm (5) auf. Dabei ist das Führungselement (10) um die Längsachse des Armgliedes (6) drehbar angeordnet. Dadurch wird in vorteilhafter Weise eine Beschädigung der Verkleidung des Roboterarms (5) durch Scheuern der Versorgungsleitungen vermieden. Weiterhin wird einer Beschädigung der Versorgungsleitungen aufgrund der reduzierten Zugkräfte auf die Versorgungsleitungen vorgebeugt.

## Beschreibung

Die vorliegende Erfindung betrifft eine Anordnung mit einem Roboter mit einem aus Armgliedern gebildeten Roboterarm, der ein von mindestens einer elektrischen Versorgungsleitung gespeistes Gerät trägt.

Ein generelles Problem bei Robotern, beispielsweise in der Ausprägung als 6-Achs-Roboter, in Industrie und Medizintechnik entsteht dann, wenn ein vom Roboterarm getragenes Gerät durch eine größere Anzahl elektrischer, hydraulischer, pneumatischer und/oder sonstiger Versorgungsleitungen versorgt wird. Diese können dann regelmäßig nicht innerhalb des Roboterarms geführt werden, da für jede Versorgungsleitung in jedem Gelenk des Roboterarms entsprechende bewegliche Durchführungen vorzusehen wären. Daher werden die Versorgungsleitungen regelmäßig außerhalb des Roboterarms zum Gerät geführt. Um diese Versorgungsleitungen vor Beschädigungen zu schützen, werden die Versorgungsleitungen häufig in einem Schlauch angeordnet. Hierfür werden bevorzugt Rillschläuche verwendet, da diese bei hoher Festigkeit eine hohe Flexibilität in alle Richtungen aufweisen.

Die Zuführung der Kabel zu der an den Roboter angebauten Peripherie muss nahe am Arm des Roboters erfolgen. Frei im Raum hängende Kabel sind nicht zulässig, um eine gefahrlose und störungsfreie Behandlung des Patienten zu gewährleisten. Weiter wird das Problem durch große Bewegungsbereiche des Roboters, durch große Durchmesser der Kabel bzw. der Kabelpakete und einzuhaltende Mindestbiegeradien der Kabel erschwert. Damit die Bewegungsfreiheit der Anordnung nicht eingeschränkt wird, muss ein bestimmter Leitungs- bzw. Schlauchvorrat vorgesehen werden. Gleichzeitig muss die Leitungs- oder Schlauchführung so erfolgen, dass der Schlauch und die im Schlauch verlaufenden Versorgungsleitungen während der Bewegung des Roboterarms und des vom Roboter getragenen Geräts nicht beschädigt werden und dass der den Bewegungen des Roboterarms mit trägheitsbedingter Verzögerung folgende Schlauch seinerseits keine Schäden an anderen Komponenten verursacht.

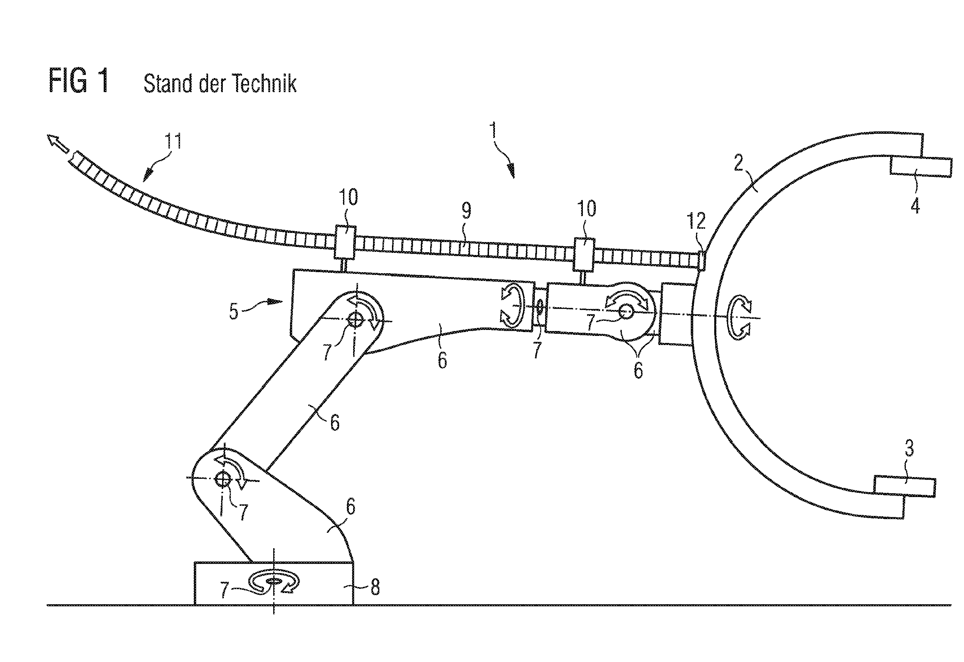

In Figur 1 ist eine Anordnung zur Führung von Versorgungsleitungen bei Robotern gemäß Stand der Technik gemäß der nachveröffentlichten Schrift 10 2009 043 448.8 dargestellt. Gezeigt ist ein C-Bogen-Röntgensystem 1, das einen C-Bogen 2 mit einer Strahlungsquelle 3 und einem Strahlungsdetektor 4 umfasst. Der C-Bogen 2 ist an einem Roboterarm 5 angeordnet, der mehrere Armglieder 6 aufweist, die über Drehgelenke 7 miteinander sowie mit dem C-Bogen 2 und einem Roboterstativ 8 verbunden sind. Die auf dem C-Bogen 2 angeordnete Strahlungsquelle 3 und der Strahlungsdetektor 4 werden über Versorgungsleitungen (nicht dargestellt) versorgt, die in einem Schlauch 9 zum C-Bogen 2 geführt werden. Durch den Schlauch 9 erfolgen einerseits eine Bündelung der Versorgungsleitungen einerseits, sowie andererseits ein Schutz vor äußeren Einflüssen und Beschädigungen. Der Schlauch 9 wird durch Führungselemente 10 entlang einzelner Armglieder 6 geführt und durch die Führungselemente 10 nahe bei dem jeweiligen Armglied 6 gehalten, so dass ein Armglied 6 und der entsprechende Abschnitt des Schlauches 9 stets korrespondierende Bewegungen ausführen und Kollisionen nicht auftreten können.

Die Führungselemente 10 sind dabei so gestaltet, dass der Schlauch 9 in Längsrichtung durch die Führungselemente 10 gleiten kann, falls durch eine Bewegung der Armglieder 6 oder des C-Bogens 2 zusätzlich Schlauch 9 benötigt oder Schlauch 9 wieder freigegeben wird. Die Führungselemente 10 können drehbar und/oder gelenkig gelagert sein, beispielsweise auf einem Kugelgelenk mit Begrenzungsmanschette, um eine Ausrichtung der Führungselemente 10 entlang einer Schlauchspannrichtung zu ermöglichen. So kann verhindert werden, dass der Schlauch 9 an den Führungselementen 10 zu stark geknickt wird.

Ein an der nicht dargestellten Decke eines Behandlungsraums, in dem sich das C-Bogen-Röntgensystem 1 befindet, montiertes Speicherelement 11 nimmt den zur Gewährleistung der Bewegungsfreiheit notwendigen Schlauchvorrat nebst Versorgungsleitungsvorrat auf und gibt ihn bei Bedarf in Abhängigkeit der von dem Roboterarm 5 durchgeführten Bewegungen frei. Das Speicherelement 11 gibt den Schlauch 9 frei, wenn die Zugbelastung des Schlauches 9 durch eine Bewegung des Roboterarms 5 zunimmt, und zieht den Schlauch 9 wieder ein, wenn die Zugbelastung des Schlauches 9 durch eine Bewegung des Roboterarms 5 abnimmt. Optional kann das Speicherelement auch am Roboterarm 5 direkt angebracht sein. Der Schlauch 9 endet am C-Bogen 2 in einem Versorgungsleitungseinlass 12. Freigabe und Einzug des Schlauches 9 können beispielsweise durch ein nicht dargestelltes motorgesteuertes Speicherrad erfolgen, das eine Gegenkraft zur Zugkraft am Schlauch 9 aufwendet, um den Schlauch 9 stets gespannt zu halten.

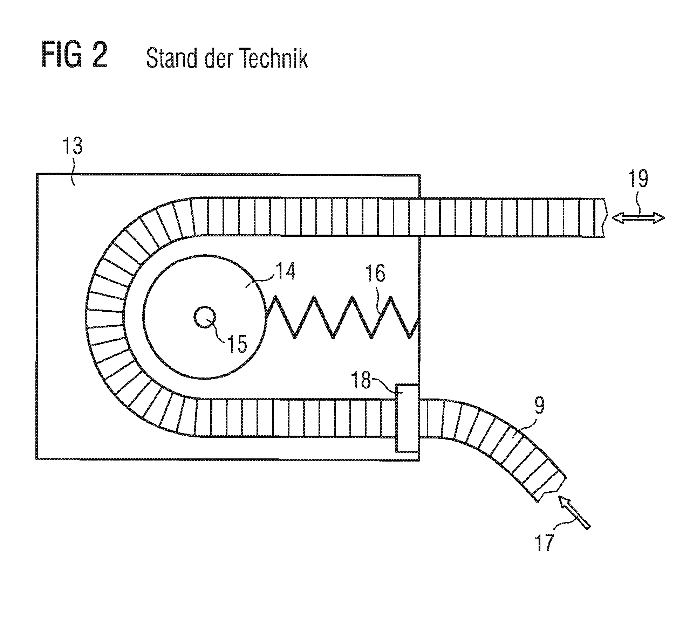

Anstelle des Speicherrads kann das Speicherelement 11 auch ein Stützrad mit einer verfahrbaren Achse umfassen. Figur 2 zeigt ein Speicherelement für Versorgungsleitungen mit einem Rad mit einer verfahrbaren Achse gemäß Stand der Technik. In einem Gehäuse 13 ist ein Stützrad 14 mit einer verfahrbaren Achse 15 angeordnet. An dem Gehäuse 13 ist eine Feder 16 befestigt, an deren anderem Ende die Achse 15 angeordnet ist. Um das Stützrad 14 ist ein Schlauch 9 geführt, der die Versorgungsleitungen umhüllt. Der Schlauch ist über eine Fixierungsschelle 18 am Gehäuse 13 befestigt. Die Zuführung des Schlauches 9 erfolgt in der angezeigten Richtung 17. Eine Veränderung der Feder 16 durch eine ausgeübte Zugkraft führt zu einer Veränderung der Position der Achse 15 und damit zu einer Veränderung der Länge des sich im Gehäuse 13 befindlichen Anteils des Schlauches 9. Nimmt die Zugbelastung des Schlauches 9 durch eine Bewegung eines nicht gezeigten Roboterarms zu, so führt dies zu einem Zusammenziehen der Feder 16, wodurch sich die Länge des Anteils des Schlauches 9 im Gehäuse verkürzt. Dadurch steht ein zusätzlicher Anteil des Schlauches 9 für die Bewegung des Roboterarms zur Verfügung. Nimmt die Zugbelastung des Schlauches 9 durch eine Bewegung des Roboterarms ab, so führt dies zu einer Entspannung der Feder 16, wodurch sich die Länge des Anteils des Schlauches 9 im Gehäuse vergrößert. Dadurch verkürzt sich der Anteil des Schlauches 9, der für die Bewegung des Roboterarms zur Verfügung steht. Bezugszeichen 19 zeigt die Richtung der Schlauchbewegung bei Bewegung des Roboterarms an.

Nachteilig an der bekannten Anordnung zur Führung der Versorgungsleitungen ist, dass der Schlauch inkl. der Versorgungsleitungen einer starken Zug- und Torsionsbelastung ausgesetzt ist. Eine Schädigung von Schlauch und Versorgungsleitungen durch diese Dauerbelastung kann nicht gänzlich vermieden werden.

Es ist Aufgabe der vorliegenden Erfindung, diese Nachteile zu überwinden und eine verbesserte Roboteranordnung anzugeben.

Diese Aufgabe wird gelöst durch eine Anordnung mit einem Roboter mit einem aus Armgliedern gebildeten Roboterarm, der ein von mindestens einer elektrischen Versorgungsleitung gespeistes Gerät trägt. Die Anordnung weist mindestens ein am Armglied befestigtes Führungselement zur Führung der Versorgungsleitung am Roboterarm auf. Dabei ist das Führungselement um die Längsachse des Armgliedes drehbar angeordnet. Dadurch erfolgt bei einer Drehung eines oder mehrerer Armglieder eine drehbare Führung der Versorgungsleitung um die Armglieder, wodurch eine Beschädigung der Verkleidung des Roboterarms durch Scheuern der Versorgungsleitung vermieden wird. Weiterhin wird einer Beschädigung der Versorgungsleitung aufgrund der reduzierten Zugkräfte auf die Versorgungsleitung vorgebeugt.

In einer besonderen Ausführungsform kann die Versorgungsleitung in einem Schlauch angeordnet sein. Vorteilhaft daran ist, dass dadurch die Versorgungsleitung vor Beschädigungen geschützt wird. Hierfür werden bevorzugt Rillschläuche verwendet, da diese bei hoher Festigkeit eine hohe Flexibilität in alle Richtungen aufweisen.

In einer vorteilhaften Ausführung kann die Anordnung mindestens ein Speicherelement zur flexiblen Aufnahme und Freigabe der Versorgungsleitung aufweisen.

In einer Weiterbildung der Erfindung kann das Speicherelement am Roboterarm angeordnet sein. Dadurch werden in vorteilhafter Weise die starken Zug- und Torsionsbelastungen auf die Versorgungsleitung bzw. auf den Schlauch reduziert und die Gefahr der Schädigung der Versorgungsleitung bzw. des Schlauches vermindert.

Weiterhin kann das Speicherelement an der Decke eines Raumes, in dem sich der Roboter befindet, angeordnet sein.

In einer weiteren Ausbildung kann das Speicherelement ein Rad mit einer eine Rückstellkraft ausübenden Drehfeder umfassen. Der Versorgungsleitungs- bzw. Schlauchvorrat wird auf einem Außendurchmesser des Rades abgelegt. Die Drehfeder sorgt in vorteilhafter Weise für eine stete Spannung der Versorgungsleitung bzw. des Schlauches.

In vorteilhafter Weise kann das Speicherelement ein Stützrad mit einer verfahrbaren Achse umfassen.

In einer Weiterbildung der Erfindung ist die verfahrbare Achse federbelastet. Durch die Federbelastung der verfahrbaren Achse ist es möglich, bei zunehmender Zugbelastung des Schlauches zusätzliche Schlauchanteile aus dem Speicherelement für Bewegungen des Roboterarms bereitzustellen und bei abnehmender Zugbelastung des Schlauches nicht mehr für Bewegung des Roboterarms benötigte Schlauchanteile in das Speicherelement einzuziehen.

Die Erfindung gibt auch ein C-Bogen-Röntgensystem mit einer Anordnung mit einem Roboter mit einem aus Armgliedern gebildeten Roboterarm, der ein von mindestens einer elektrischen Versorgungsleitung gespeistes Gerät trägt, an. Die Anordnung weist mindestens ein am Armglied befestigtes Führungselement zur Führung der Versorgungsleitung am Roboterarm auf. Dabei ist das Führungselement um die Längsachse des Armgliedes drehbar angeordnet.

Weitere Besonderheiten und Vorteile der Erfindung werden aus den nachfolgenden Erläuterungen mehrerer Ausführungsbeispiele anhand von schematischen Zeichnungen ersichtlich.

Es zeigen:
- Figur 1:: eine Anordnung zur Führung von Versorgungsleitungen bei Robotern gemäß Stand der Technik,
- Figur 2:: ein Speicherelement für Versorgungsleitungen mit einem Rad mit einer verfahrbaren Achse gemäß Stand der Technik, und
- Figur 3: eine Anordnung zur Führung von Versorgungsleitungen mit drehbaren Führungselementen bei Robotern.

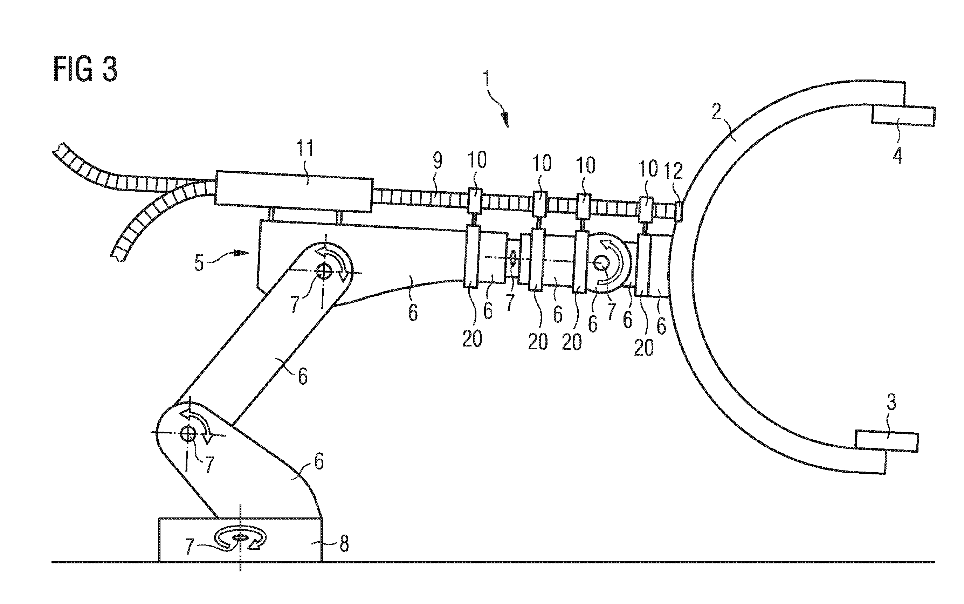

Figur 3 zeigt eine Anordnung zur Führung von Versorgungsleitungen mit drehbaren Führungselementen bei Robotern. Dargestellt ist ein C-Bogen-Röntgensystem 1, das einen C-Bogen 2 mit einer Strahlungsquelle 3 und einem Strahlungsdetektor 4 umfasst. Der C-Bogen 2 ist an einem Roboterarm 5 angeordnet, der mehrere Armglieder 6 aufweist, die über Drehgelenke 7 miteinander sowie mit dem C-Bogen 2 und einem Roboterstativ 8 verbunden sind. Die auf dem C-Bogen 2 angeordnete Strahlungsquelle 3 und der Strahlungsdetektor 4 werden über Versorgungsleitungen (nicht dargestellt) versorgt, die in einem Schlauch 9 zum C-Bogen 2 geführt werden. Ein an dem Roboterarm 5 angebrachtes Speicherelement 11 nimmt den zur Gewährleistung der Bewegungsfreiheit notwendigen Schlauchvorrat nebst Versorgungsleitungsvorrat auf und gibt ihn bei Bedarf in Abhängigkeit der von dem Roboterarm 5 durchgeführten Bewegungen frei. Das Speicherelement 11 gibt den Schlauch 9 frei, wenn die Zugbelastung des Schlauches 9 durch eine Bewegung des Roboterarms 5 zunimmt, und zieht den Schlauch 9 wieder ein, wenn die Zugbelastung des Schlauches 9 durch eine Bewegung des Roboterarms 5 abnimmt. Der Schlauch 9 endet am C-Bogen 2 in einem Versorgungsleitungseinlass 12. Freigabe und Einzug des Schlauches 9 können beispielsweise durch ein nicht dargestelltes motorgesteuertes Speicherrad erfolgen, das eine Gegenkraft zur Zugkraft am Schlauch 9 aufwendet, um den Schlauch 9 stets gespannt zu halten.

Der Schlauch 9 wird durch Führungselemente 10 entlang einzelner Armglieder 6 geführt. Ein Führungselement 10 ist dabei so gestaltet, dass der Schlauch 9 in Längsrichtung durch das Führungselement 10 gleiten kann, falls durch eine Bewegung der Armglieder 6 oder des C-Bogens 2 zusätzlich Schlauch 9 benötigt oder Schlauch 9 wieder freigegeben wird. Das Führungselement 10 kann in sich drehbar und/oder gelenkig gelagert sein, beispielsweise auf einem Kugelgelenk mit Begrenzungsmanschette, um eine Ausrichtung des Führungselementes 10 entlang einer Schlauchspannrichtung zu ermöglichen. So kann verhindert werden, dass der Schlauch 9 an einem Führungselement 10 zu stark geknickt wird.

Erfindungsgemäß ist ein Führungselement 10 beweglich verfahrbar in einer Führungsschiene 20 angeordnet, die kreisförmig ein Armglied 6 umgibt. Dadurch ist das Führungselement 10 beweglich verfahrbar um die Längsachse eines Armglieds 6 angeordnet, wodurch eine flexible Führung des Schlauches 9 um den Roboterarm 5 hergestellt wird. Bei einer Drehung eines oder mehrerer Armglieder 6 erfolgt eine drehbare und bewegliche Führung des Schlauches 9 bzw. der Versorgungsleitungen um die Armglieder 6, wodurch eine Beschädigung der Verkleidung des Roboterarms 5 durch Scheuern des Schlauches 9 vermieden wird. Weiterhin wird einer Beschädigung des Schlauches 9 aufgrund der reduzierten Zugkräfte auf den Schlauch 9 vorgebeugt. Die Führungselemente 20 können an der Verkleidung der Armglieder 6 angebracht oder in die Verkleidung integriert sein.

### Bezugszeichenliste

- 1: C-Bogen-Röntgensystem
- 2: C-Bogen
- 3: Strahlungsquelle
- 4: Strahlungsdetektor
- 5: Roboterarm
- 6: Armglieder
- 7: Drehgelenke
- 8: Roboterstativ
- 9: Schlauch
- 10: Führungselemente
- 11: Speicherelement
- 12: Versorgungsleitungseinlass
- 13: Gehäuse
- 14: Stützrad
- 15: Achse
- 16: Feder
- 17: Zuführungsrichtung Schlauch
- 18: Fixierungsschelle
- 19: Richtung der Schlauchbewegung
- 20: Führungsschiene

## Patentansprüche

1. Anordnung mit
- einem Roboter (1) mit einem aus Armgliedern (6) gebildeten Roboterarm (5), der ein von mindestens einer elektrischen Versorgungsleitung gespeistes Gerät (3,4) trägt,
- mindestens einem am Armglied (6) befestigten Führungselement (10) zur Führung der Versorgungsleitung am Roboterarm (5),
**dadurch gekennzeichnet,**
**dass** das Führungselement (10) um die Längsachse des Armgliedes (6) drehbar angeordnet ist.

2. Anordnung nach Anspruch 1,
**dadurch gekennzeichnet,**
**dass** die Versorgungsleitung in einem Schlauch (9) angeordnet ist.

3. Anordnung nach Anspruch 1 oder 2,
**gekennzeichnet durch**:
- mindestens ein Speicherelement (11) zur flexiblen Aufnahme und Freigabe der Versorgungsleitung.

4. Anordnung nach Anspruch 3,
**dadurch gekennzeichnet,**
**dass** das Speicherelement (11) am Roboterarm (5) angeordnet ist.

5. Anordnung nach einem der Ansprüche 3 oder 4,
**dadurch gekennzeichnet,**
**dass** das Speicherelement (11) an der Decke eines Raumes, in dem sich der Roboter befindet, angeordnet ist.

6. Anordnung nach einem der Ansprüche 3 bis 5,
**dadurch gekennzeichnet,**
**dass** das Speicherelement (11) ein Rad mit einer eine Rückstellkraft ausübenden Drehfeder umfasst.

7. Anordnung nach einem der Ansprüche 3 bis 6,
**dadurch gekennzeichnet,**
**dass** das Speicherelement ein Stützrad (14) mit einer verfahrbaren Achse (15) umfasst.

8. Anordnung nach Anspruch 7,
**dadurch gekennzeichnet,**
**dass** die Achse federbelastet ist.

9. C-Bogen-Röntgensystem mit einer Anordnung nach einem der vorherigen Ansprüche.
